# EUROPÄISCHE PATENTANMELDUNG

(11) **EP 1 041 159 A2**
(43) Veröffentlichungstag der Anmeldung: **04.10.2000**
(21) Anmeldenummer: 00106583.8
(22) Anmeldetag: 27.03.2000
(51) Int. Cl.: C12Q 1/68

(54) **Verfahren, Oligonucleotide zum Abbau von in-vitro synthetisierten Nucleinsäuremolekülen**

(30) Priorität: 26.03.1999 EP 99106315; 26.03.1999 DE 19913834
(71) Anmelder: MIRA Diagnostika GmbH, 51377 Leverkusen (DE)
(72) Erfinder: Leiser, Robert-Matthias, 42697 Solingen (DE); Plobner, Lutz, 40699 Erkrath (DE); Temper, Jochen, 51375 Leverkusen (DE); Zavriev, Sergei Kiriakovich, 125422 Moskau (RU); Alexseev, Jakov Igorevich, 121596 Moskau (RU)
(74) Vertreter: Meyers, Hans-Wilhelm, Dr.Dipl.-Chem.

(57) **Zusammenfassung**

Verfahren zum Abbau von in-vitro synthetisierten Nucleinsäuremolekülen mit den Schritten
- Einbau von mindestens einem Derivat von Nucleotidresten in mindestens ein für die Amplifikation notwendiges Starter-Oligonucleotid,
- Durchführung der Amplifikation und Einbau der/des Starteroligonucleotide(s) in die Amplifikationsprodukte und nach Beendigung der Amplifikation
- Abbau der Starter-Oligonucleotid-Sequenzen und deren Komplementärsequenzen,
   wobei der Abbau der Starter-Oligonucleotid-Sequenzen und deren Komplementärsequenzen folgende Schritte umfasst:
- Transformation des mindestens einen Derivates von Nucleotidresten, wodurch dieses zum Substrat für eine Nucleinsäureglycosylase wird,
- Abspaltung der Base des mindestens einen Derivates mittels einer Nucleinsäureglycosylase, die das mindestens eine Derivat erkennt,
- Schaffung von Strangbrüchen an der (den) Stelle(n), an der (denen) die Base(n) abgespalten worden ist (sind),
- Trennung der/des durch die Abspaltung entstandenen Oligonucleotide(s) von den Komplementärsträngen der Amplifikationsprodukte und
- Entfernung (Hydrolyse) des (der) entstandenen überhängenden 3*'*-Endes (en) der Amplifikationsprodukte durch eine Nuclease, die 3*'*-überhängende Enden eines Nucleinsäurestranges abspalten kann.

## Beschreibung

Gegenstand der vorliegenden Erfindung sind Verfahren und Oligonucleotide zum Abbau von in-vitro synthetisierten Nucleinsäuremolekülen.

US-A-5,035,996 und US-A-5,683,896 (Life Technologies) offenbaren Kontaminationen in Reaktionsansätzen zur DNA-Amplifikation dadurch zu kontrollieren, dass in die verwendeten Starter-Oligonucleotide bestimmte Derivate von Nucleotidresten (z.B. Desoxyuridin) eingebaut werden. Vor einer neuen Amplifikationsreaktion können dann eventuell im Reaktionsansatz befindliche Amplifikationsprodukte aus vorangegangenen Amplifikationen dadurch entfernt werden, dass durch Verwendung von geeigneten Enzymen (z.B. Uracil-DNA-Glycosylase, UDG) Strangbrüche in den Amplifikationsprodukten an der Stelle erzeugt werden, an der sich bei den vorangegangenen Amplifikationen die Derivate von Nucleotidresten in den Starter-Oligonucleotiden befanden.

WO 92/01814 (Cetus) als auch WO 97/12061 offenbaren, ein Nucleotidderivat bei der DNA-Synthese in Form des Triphosphats anstelle eines normalen dNTP's (z.B. dUTP anstelle dTTP) randomisiert einzubauen. Vor einer neuen Amplifikation wird verfahren wie in den oben genannten Life Technologies Veröffentlichungen.

Das Verfahren nach Life Technologies hat gegenüber WO 92/01814 den Vorteil, dass durch den gezielten Einbau der Nucleotidderivate in die Starter-Oligonucleotide die UDG nicht das gesamte Amplifikat zerstört, sondern nur die Abschnitte abgetrennt werden, die nötig sind, damit in einer neuen Amplifikationsreaktion die DNA durch neue Starter-Oligonucleotide nicht erkannt wird. Dies hat insbesondere den Vorteil, dass man entstandene Amplifikate so verändern kann, dass sie nicht mehr als Vorlage (Template) für weitere Amplifikationen dienen können, jedoch immer noch detektierbar bleiben. Ein zweiter Vorteil besteht darin, dass die häufig auftretende partielle Syntheseinhibition und der dadurch bedingte Sensitivitätsverlust als Folge des zumindest teilweisen Ersatzes des dTTP durch dUTP umgangen wird.

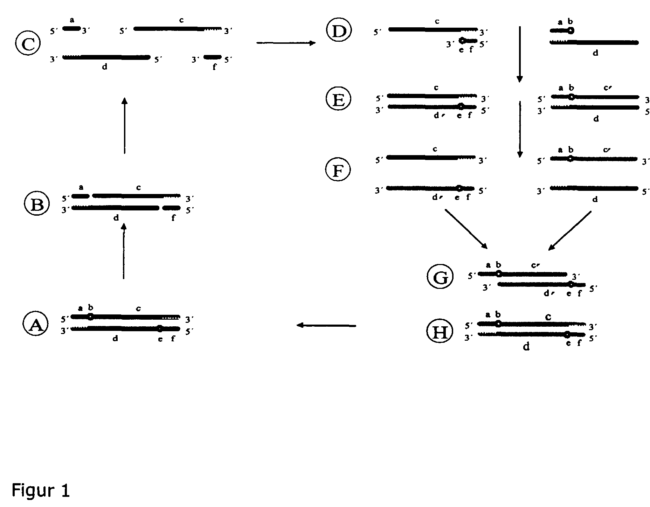

Eigene Untersuchungen haben ergeben, dass das Verfahren nach US-A-5,035,996 und US-A-5,683,896 nicht funktionell ist. Ein wichtiger Grund für das Fehlschlagen des Verfahrens besteht in folgendem: Die Nucleotidderivate werden als Bestandteil der Starter-Oligonucleotide jeweils in der Nähe der 5*'*-Enden der Amplifikationsprodukte eingebaut. Durch Strangbruch und Abtrennung dieses Oligonucleotids werden aber eventuelle Kontaminationen in neuen Amplifikations-Reaktionsansätzen nicht wirksam zerstört, weil zwar jeweils die 5*'*-Enden des Amplifikationsproduktes gekürzt werden, diese aber durch mehrere Schritte von Denaturierung, Renaturierung und Strangverlängerung, wie sie bei erneuter PCR ablaufen, wieder regeneriert werden können. Dies veranschaulicht Figur 1.

Weitere Gründe des Fehlschlagens dieses Verfahrens können unter bestimmten Bedingungen darin bestehen, dass UDG zwar die Stickstoffbase an der Stelle des Nucleotidderivat-Einbaus angreift, d.h. einen sogenannten abasischen Lokus kreiert, aber dadurch noch keinen Strangbruch verursacht (David und Williams, 1998; Krokan et al., 1997). Dies veranschaulicht Figur 2. Zur wirksamen, sicheren Erzeugung von Strangbrüchen nach Einwirkung von UDG wird z.B. in WO 97/12061 eine ganze Reihe von Möglichkeiten angeführt.

Das der Erfindung zu Grunde liegende technische Problem besteht insbesondere darin, ein Verfahren zur Kontaminationskontrolle von Amplifikationsprozessen zu schaffen, mit dem amplifizierte Nukleinsäurestränge nach Amplifikation so modifiziert werden, dass sie nicht länger als Template einer neuen Amplifikation, jedoch immer noch zur Detektion der ersten Amplifikation dienen können. Hierbei werden die Mängel der US-A-5,035,996 und US-A-5,683,896 überwunden. Die Besonderheit des erfindungsgemäßen Verfahrens besteht darin, dass in mindestens eines der verwendeten Primer-Oligonucleotide mindestens ein Nucleotidderivat eingebaut wird, das durch Transformation zu einem gewünschten Zeitpunkt in ein Derivat überführt wird, das Substrat für eine DNA-Glykosylase ist und damit durch diese Glykosylase angegriffen wird. An der Angriffstelle entsteht durch nachfolgende allgemein bekannte Prozesse jeweils ein Strangbruch. In einer bevorzugten Variante der Erfindung wird ein Verfahren vorgestellt, das es erlaubt, das für die Dekontamination dienende Enzym bereits zum ersten Amplifikationsgemisch hinzuzugeben, ohne dass dieses die eingefügten Derivate von Nucleotidresten als Substrat erkennt. Erst nach Abschluss der Amplifikation wird durch Transformation der Derivate von Nucleotidresten deren Abbau durch das dekontaminierende Enzym möglich. In einer bevorzugten Variante wird 3-Methyladenin-DNA-Glykosylase II als Enzym zum Abbau der nach Transformation umgeformten Derivate, konkret von 5-Formyl-Desoxy-uridinresten verwandt. Im Unterschied zu allen Methoden (z.B. Ono etal, Nucleosides and Nucleotides. 131. "Synthesis and properties of oligonucleotides containing 5-formyl-2*'*-deoxyuridine", in Chemical and Pharmaceutical bulletins, Bd. 42, Nr. 11, 1994, 2231-2237; Sugiyama et al., "New Synthetic method of 5-formyluracil containing oilgonucleotides and their melting behavior" Tetrahedron Letters, Bd. 37, 1996, 9067-9070; Zhang et al., "Replication of DNA templates containing 5-formyluracil...", Nucleic Acids Research, Bd 25, 1997, 3969-3973), die den Einbau von Formyl-Desoxyuracil in Polynucleotide für verschiedene Zwecke beschreiben, zielt die vorliegende Erfindung nicht auf den Einbau von Formyl-uracyl-Derivaten, sondern auf den Einbau von an der Seitenkette halogenierten Nucleosidresten in die Primer-Oligonucleotide, damit an diesen Stellen zum gewünschten Zeitpunkt nach Transformation in die entsprechenden Formylderivate Kettenbrüche erzeugt werden können.

In DD/265429 wurde die Verwendung von an Seitenketten halogenierten Nukleosiden mit dem Ziel einer nichtradioaktiven Markierung von Polynucleotiden beschrieben, die nach Einbau in Polynucleotide in entsprechende Formylverbindungen umgeformt wurden. Diese reaktiven Formylverbindungen dienten dann zur Kopplung von Markerbausteinen für die nichtradioaktive Markierung. Der besondere Vorteil dieses Verfahren bestand darin, dass die halogenierten Verbindungen besser in die Polynucleotide eingebaut wurden als die späteren Formylverbindungen (wie Formyl-Desoxyuridin). Im Unterschied zu DD/265429 werden die halogenierten Nukleoside und deren spätere Umwandlung in die Formylverbindungen mit völlig anderer, nicht naheliegender Zielsetzung eingesetzt, nämlich nicht mit dem Ziel der Kopplung eines Markerbausteins nach erfolgter Transformation, sondern ihres Abbaus mit einer speziell passenden Glykosylase für die nach Transformation entstehende Formylverbindung. Der entscheidende Vorteil dieser Variante gegenüber allen bisher bekannten Verfahren zur Dekontamination von Amplifikationsprozessen besteht darin, dass der Dekontaminationsprozeß im Reaktionsgemisch der ersten Amplifikationsreaktion stattfinden kann, d.h. bevor überhaupt Kontaminationen erzeugt werden können.
Figur 1: Regenerierung der abgeschnittenen Primersequenzen.
   A bis C: Behandlung der in der ersten PCR entstandenen Amplifikate mit UDG
   D bis F: Synthese von neuen Amplifikaten während der zweiten PCR an den Templates der durch UDG-Hydrolyse modifizierten Amplifikate aus der ersten PCR
   G bis H: Hybridisierung der in der zweiten PCR am Template der modifizierten Amplifikate aus der ersten PCR synthetisierten Amplifikatstränge miteinander und Regenerierung der kompletten ursprünglichen Amplifikatsequenzen durch Auffüllreaktion
   A: Amplifikat mit eingebauten Starteroligonucleotiden, die Desoxyuridinrest(e) (dU) enthalten
   B: Amplifikat nach Abbau von dU und Strangbruch
   C: Trennung der Amplifikat-DNA(reste) und der durch dU-Abbau und Strangbruch abgetrenneten Starteroligonucleotid(reste)
   D: Hybridisierung zwischen den Amplifikat-DNA(resten) und neuen Starteroligonucleotiden
   E: DNA Neusynthese am Template der Amplifikat-DNA(reste) (Auffüllreaktion)
   F: Denaturierung der DNA-Doppelstränge
   G: Hybridisierung zwischen den neu synthetisierten komplementären Nucleinsäuresträngen
   H: DNA-Neusynthese (Auffüllreaktion) und damit komplette Regenerierung der in der ersten PCR erzeugten Amplifikatsequenzen.

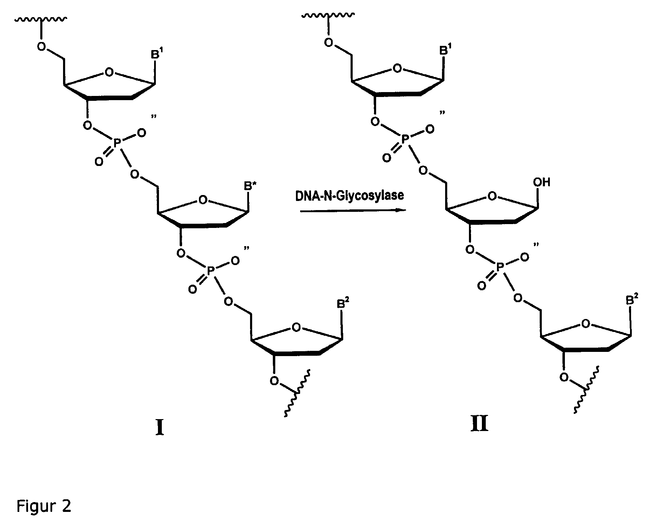

Figur 2: Glycosylaseschritt: Abspaltung des Uracilrestes vom DNA-Strang.
Figur 3: Erzeugung eines DNA-Strangbruches und Schaffung von 3*'*-OH-Enden.
Figur 4: Wirkungsmechanismus einer Exonuclease (links, z.B. Exo III) und einer Endonuclease (rechts, z.B. Endo IV) auf überstehende Stränge

Das erfindungsgemäße Verfahren zum Abbau von in-vitro synthetisierten Nucleinsäuremolekülen weist im Einzelnen die folgenden Schritte auf:
- Einbau von mindestens einem Derivat von Nucleotidresten in mindestens ein für die Amplifikation notwendiges Starter-Oligonucleotid,
- Durchführung der Amplifikation und Einbau der/des Starter-Oligonucleotide(s) in die Amplifikationsprodukte und nach Beendigung der Amplifikation
- Abbau der Starter-Oligonucleotid-Sequenzen und deren Komplementärsequenzen,
   wobei der Abbau der Starter-Oligonucleotid-Sequenzen und deren Komplementärsequenzen folgende Schritte umfasst:
- Transformation des mindestens einen Derivates von Nucleotidresten, wodurch dieses zum Substrat für eine Nucleinsäureglycosylase wird,
- Abspaltung der Base des mindestens einen Derivates mittels einer Nucleinsäureglycosylase, die das transformierte mindestens eine Derivat erkennt,
- Schaffung von Strangbrüchen an der (den) Stelle(n), an der (denen) die Base(n) abgespalten worden ist (sind),
- Trennung der/des durch die Abspaltung entstandenen Oligonucleotide(s) von den Komplementärsträngen der Amplifikationsprodukte und
- Entfernung (Hydrolyse) des (der) entstandenen überhängenden 3*'*-Endes (en) der Amplifikationsprodukte durch eine Nuclease, die 3*'*-überhängende Enden eines Nucleinsäurestranges abspalten kann.

In dem erfindungsgemäßen Verfahren werden vorzugsweise als Amplifikationsprodukte in-vitro synthetisierte Nucleinsäuremoleküle verwendet.

Die Starter-Oligonucleotide weisen vorzugsweise mindestens eine Struktureinheit auf, die mindestens eine Erkennungsstelle für Glycosylasen aufweist, die während der Oligonucleotidsynthese und Amplifikation durch eine transformierbare Gruppe modifiziert ist, die nach einer Transformation die Erkennungsstelle freilegt.

Als Transformation wird hier eine Abspaltungsreaktion verstanden, die chemisch, insbesondere photolytisch, oder biochemisch, insbesondere enzymatisch ist.

Die Struktureinheit im erfindungsgemäßen Oligonucleotid ist ein atypischer Nucleosidrest, der aus einer atypischen Base, die C-N-glycosidisch mit einem Zuckerbaustein der Gruppe der Pentosen und Hexosen bzw. Desoxypentosen und Desoxyhexosen verknüpft ist.

Als "atypische Base" werden in den erfindungsgemäßen Oligonucleotiden die folgenden Verbindungen verstanden: 5-(2-(1,3-Dioxanyl))-uracil, 5-(2-Nitrobenz-oxy)methylcytosin, 5-(4-Methoxy-2-nitrobenzoxy)methylcytosin, 5-(4, 5- Dimetho-xy-2-nitrobenzoxy)methylcytosin [= 5-(6-Nitroveratryloxy)methylcytosin], 5-(2-Nitrobenzoxy)methyluracil, 5-(4-Methoxy-2-nitrobenzoxy)methyluracil, 5-(4,5-Dimethoxy-2-nitrobenzoxy)methyluracil [= 5-(6-Nitroveratryloxy)methyluracil], 5-(2-(4-(2-Nitrophenyl)-1,3-dioxolanyl))methyluracil, 6-(2-Nitrobenzoxy)purin, 6-(4-Methoxy-2-nitrobenzoxy)purin, 6-(4,5-Dimethoxy-2-nitrobenzoxy)purin [= 5-(6-Nitroveratryloxy)purin].

Durch Transformation wird die atypische Base insbesondere in Hypoxanthin, 5-Formyluracil, 5-Hydroxymethylcytosin, 5-Hydroxymethyluracil umgeformt und wird so zur Glycosylase-Erkennungsstelle.

Vorzugsweise handelt es sich bei der Nucleinsäureglycosylase um 3-Methyladenin-DNA-Glykosylase II, 5-Hydroxymethylcytosin-DNA-Glycosylase, Formamidopyrimidin-DNA-Glycosylase, 5-Hydroxymethyluracil-DNA-Glycosylase und Hypoxanthin-DNA-Glycosylase. Vorzugsweise handelt es sich bei der verwendeten Glycosylase um ein thermostabiles Enzym.

Die Zugabe der Nucleinsäureglycosylase zum Abbau der Starter-Oligonucleotid-Sequenzen und deren Komplementärsequenzen kann beispielsweise zum Reaktionsgemisch vor Beginn der neuen Amplifikation erfolgen.

Die Zugabe der Nucleinsäureglycosylase zum Abbau der Starter-Oligonucleotid-Sequenzen und deren Komplementärsequenzen kann vorzugsweise zum Reaktionsgemisch der ursprünglichen Amplifikation erfolgen.

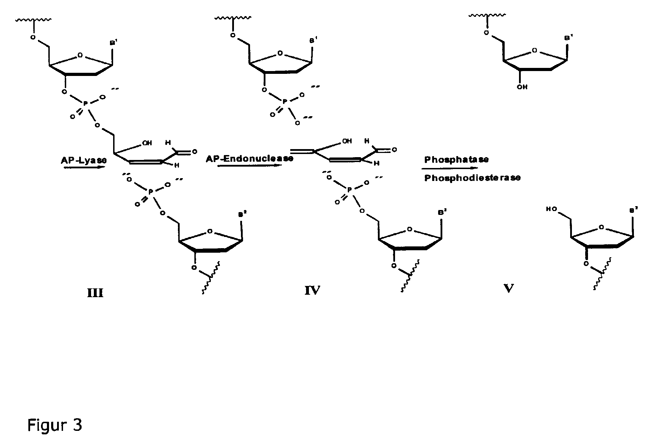

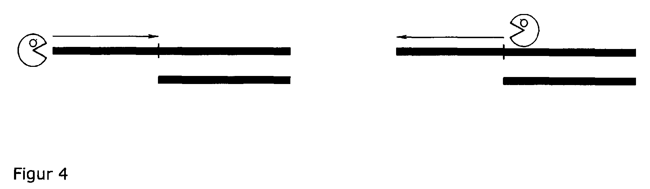

Die Erzeugung von Strangbrüchen an den durch die Glykosylase geschaffenen Halbacetalen bzw. Hydroxyaldehyden und Ablösung der dabei abgetrennten, aber noch in Basenpaarung mit dem Komplementärstrang befindlichen Oligonucleotiden kann z.B. wie in WO 97/12061 oder WO 98/38296 durch erhöhte Temperatur, durch Lyase-, Phosphatase- oder Phosphodiesterase-Aktivität erfolgen (Figur 3). Dadurch kann die zuvor genannte 3*'*-5*'*-Exonuclase sukzessive die abgetrennten, aber noch in Basenpaarung mit dem Komplementärstrang befindlichen Oligonucleotide abbauen und die dadurch entstehenden 3*'* -überhängenen DNA-Stränge angreifen. Dies veranschaulicht Figur 4.

Die notwendige 3*'*-5*'*-Exonuclease-Aktivität kann verschiedenen Ursprungs sein. Es kann z.B. eine DNA-Polymerase zur Amplifikation verwendet werden, die selbst eine solche Aktivität aufweist (z.B. Pfu-DNA-Polymerase), oder es kann eine solche DNA-Polymerase mit 3'-5'-Exonuclease-Aktivität im Gemisch z.B. mit der in PCR-Reaktionen häufig verwandten Taq- bzw. Tth-DNA-Polymerase eingesetzt werden. Schließlich kann eine 3'-5'-Exonuclease ohne eigene DNA-Polymerase-Aktivität eingesetzt werden. Bei letzteren handelt es sich insbesondere um natürliche Nucleasen (z.B. Exonuclease III, Exonuclease VII oder Endonuclease IV), die auch rekombinant gewonnen werden können, oder aber um Mutanten von DNA-Polymerasen mit 3'-5'-Exonuclease-Aktivität, bei denen die Polymeraseaktivität verändert wurde.

Die erfindungsgemäßen Oligonucleotide, die im erfindungsgemäßen Verfahren eingesetzt werden können, enthalten mindestens ein Derivat von Nucleotidresten, deren Stickstoffbase nach Transformation durch eine Nucleinsäureglycosylase abspaltbar ist.

Die transformierbare Gruppe ist vorzugsweise so gestaltet, dass sie mit einer chemischen, insbesondere photolytischen, oder biochemischen, insbesondere enzymatischen Reaktion eine Erkennungsstelle freilegt.

Die Struktureinheit im erfindungsgemäßen Oligonucleotid ist ein atypischer Nucleosidrest, der aus einer atypischen Base, die C-N-glycosidisch mit einem Zuckerbaustein der Gruppe der Pentosen und Hexosen bzw. Desoxypentosen und Desoxyhexosen verknüpft ist.

Als "atypische Base" werden in den erfindungsgemäßen Oligonucleotiden die folgenden Verbindungen verstanden: 5-(2-(1,3-Dioxanyl))-uracil, 5-(2-Nitrobenz-oxy)methylcytosin, 5-(4-Methoxy-2-nitrobenzoxy) methylcytosin, 5-(4,5-Dimetho-xy-2-nitrobenzoxy)methylcytosin [= 5-( 6-Nitroveratryloxy)methylcytosin], 5-(2- Nitrobenzoxy)methyluracil, 5-(4-Methoxy-2-nitrobenzoxy)methyluracil, 5-(4,5-Dimethoxy-2-nitrobenzoxy)methyluracil [= 5-(6-Nitroveratryloxy)methyluracil], 5-(2-(4-(2-Nitrophenyl)-1,3-dioxolanyl))methyluracil, 6-(2-Nitrobenzoxy)-purin, 6-(4-Methoxy-2-nitrobenzoxy)purin, 6-(4,5-Dimethoxy-2-nitrobenzoxy)purin [= 5-(6-Nitroveratryloxy)purin].

Durch Transformation wird die atypische Base insbesondere in Hypoxanthin, 5-Formyluracil, 5-Hydro-xymethylcytosin, 5-Hydroxymethyluracil umgeformt und wird so zur Glycosylase-Erkennungsstelle..

Die Erfindung wird anhand des folgenden Ausführungsbeispiels näher erläutert:

### Beispiel

Zur Durchführung der PCR werden folgende Reaktionskomponenten miteinander vermischt:

| | |
|---|---|
| 10x PCR-Puffer (0,1% Tween, 660mM Tris-HCl pH 8,8, 166mM (NH₄)₂SO₄ | 5,0µl |
| MgCl₂ (25mM) | 5,0µl |
| dNTP's (je 2mM) | 5,0µl |
| DNA-Polymerase (5U/µl) | 0,2µl |
| DNA-Template (0,2ng/µl) | 5,0µl |
| Primer A (30pmol) | 1,0µl |
| Primer B (30pmol) | 1,0µl |
| H₂O | 27,8µl |

Die Amplifikation der Ziel-DNA (Target-DNA) erfolgt in einem Thermocycler (GeneAmp PCR System 9700 von Perkin Elmer) mit vorzugsweise beheiztem Deckel. Hierbei wird die DNA zuerst für 5min bei 94°C denaturiert und anschließend für 30 Zyklen unter folgenden Bedingungen amplifiziert:

| | | |
|---|---|---|
| 30sec | 94°C | Zyklus-Denaturierung |
| 15sec | 56°C | Primer-Hybridisierung ("Annealing") |
| 20sec | 72°C | Elongation ("Extension") |

Zum Abschluss des PCR-Programmes erfolgt eine nochmalige Elongation bei 72°C für 1min.

### Ausführungsbeispiel 1

### Abkürzungen:

| | |
|---|---|
| AlkA | 3-Methyladenin-DNA-Glykosylase II. |
| dNVU | 2'-Desoxy-5-(6-nitroveratryloxy)methyluridin |
| dNVUCP | 5'-Dimethoxytrityl-dNVU-3'-[(2-cyanethyl)-(N,N-diisopropyl)]-phosphoramidit |

Das dNVUCP wird wie folgt synthetisiert:

Das aus 3',5'-Diacetylthymidin (via 3',5'-Diacetyl-5-brommethyl-2'-desoxyuridin) und 2-Nitroveratrylalkohl nach Hydrolyse erhältliche dNVU wird mit 4,4'-Dimethoxytriphenylchlormethan und danach mit Phosphorigsäure-mono(2-cyanethylester)diisopropylamidchlorid zum dNVUCP umgesetzt.

Die Oligonucleotide werden im Syntheseautomaten nach den dem Gerät entsprechenden Vorschriften synthetisiert, dabei muss die Kopplungsdauer des modifizierten Bausteins mindestens um den Faktor 10 verlängert werden. Der Einbau des dNVUCP erfolgt im konkreten Beispiel an jeweils drei Thymin-Positionen der Primer.

Nach einer sich anschließenden PCR (entsprechend dem vorstehenden Beispiel) wird ein Enzymgemisch aus 1 µl AlkA (1 Unit/100) und 0,5 µl Pfu-DNA-Polymerase (5 Unit/µl) zugegeben und erhitzt für 10 min bei 37°C.

### Beispiel 2

Synthese der Oligonucleotide und Durchführung der PCR erfolgt wie oben beschrieben. Nach der PCR-Amplifikation wird mit Licht geeigneter Wellenlänge bestrahlt (handelsüblicher Transilluminator mit λ = 350 bis 366 nm, 15 Minuten) und ein Enzymgemisch aus 1 µl AlkA (1 U-nit/100), 2 µl Exonuclease III (1 unit/ 100 µl) sowie 2 µl Exonuclease VII (1 unit/100 µl) zugesetzt.

Man inkubiert für 30 min bei 31°C.

## Patentansprüche

1. Verfahren zum Abbau von in-vitro synthetisierten Nucleinsäuremolekülen mit den Schritten
• Einbau von mindestens einem Derivat von Nucleotidresten in mindestens ein für die Amplifikation notwendiges Starter-Oligonucleotid,
• Durchführung der Amplifikation und Einbau der/des Starter-Oligonucleotide(s) in die Amplifikationsprodukte und nach Beendigung der Amplifikation
• Abbau der Starter-Oligonucleotid-Sequenzen und deren Komplementärsequenzen,
wobei der Abbau der Starter-Oligonucleotid-Sequenzen und deren Komplementärsequenzen folgende Schritte umfasst:
• Transformation des mindestens einen Derivates von Nucleotidresten, wodurch dieses zum Substrat für eine Nucleinsäureglycosylase wird,
• Abspaltung der Base des mindestens einen Derivates mittels einer Nucleinsäureglycosylase, die das transformierte mindestens eine Derivat erkennt,
• Schaffung von Strangbrüchen an der (den) Stelle(n), an der (denen) die Base(n) abgespalten worden ist (sind),
• Trennung der/des durch die Abspaltung entstandenen Oligonucleotide(s) von den Komplementärsträngen der Amplifikationsprodukte und
• Entfernung (Hydrolyse) des (der) entstandenen überhängenden 3*'*-Endes (en) der Ampilfikationsprodukte durch eine Nuclease, die 3*'*-überhängende Enden eines Nucleinsäurestranges abspalten kann.

2. Verfahren nach Anspruch 1, gekennzeichnet dadurch, dass es sich bei den Amplifikationsprodukten um in-vitro synthetisierte Nucleinsäuremoleküle handelt.

3. Verfahren nach Anspruch 1, gekennzeichnet dadurch, dass es sich bei den Amplifikationsprodukten um in-vitro ligierte Nucleinsäuremoleküle handelt.

4. Verfahren nach Anspruch 1 bis 3, gekennzeichnet dadurch, dass es sich bei den in die Starter-Oligonucleotide eingebauten Derivaten von Nucleotidresten um 5-(2-(1,3-Dioxanyl))-uracil, 5-(2-Nitrobenzoxy)methylcytosin, 5-(4-Methoxy-2-nitrobenzoxy)methylcytosin, 5-(4,5-Dimethoxy-2-nitrobenzoxy)methylcytosin [= 5-(6-Nitroveratryloxy)methylcytosin], 5-(2-Nitrobenzoxy)methyluracil, 5-(4-Metho-xy-2-nitrobenzoxy)methyluracil, 5-(4,5-Dimethoxy-2-nitrobenzoxy)methyluracil [= 5-(6-Nitroveratryloxy)methyluracil], 5-(2-(4-(2-Nitrophenyl)-1,3-dioxola-nyl))-methyluracil, 6-(2-Nitrobenzoxy)purin, 6-(4-Methoxy-2-nitrobenzoxy)purin, 6-(4,5-Dimethoxy-2-nitrobenzoxy)purin [= 5-(6-Nitroveratryloxy)purin] handelt.

5. Verfahren nach Anspruch 1 bis 4, gekennzeichnet dadurch, dass es sich bei der Nucleinsäureglycosylase um 3-Methyladenin-DNA-Glykosylase II, 5-Hydroxymethylcytosin-DNA-Glycosylase, Formamidopyrimidin-DNA-Glycosylase, 5-Hydroxymethyluracil-DNA-Glycosylase und Hypoxanthin-DNA-Glycosylase handelt.

6. Verfahren nach Anspruch 5, gekennzeichnet dadurch, dass es sich bei der Nucleinsäureglycosylase um ein thermostabiles Enzym handelt.

7. Verfahren nach Anspruch 1 bis 6, gekennzeichnet dadurch, dass die Transformation des mindestens einen Derivates von Nucleotidresten chemisch, insbesondere photolytisch, erfolgt.

8. Verfahren nach Anspruch 1 bis 6, gekennzeichnet dadurch, dass die Transformation des mindestens einen Derivates von Nucleotidresten biochemisch, insbesondere enzymatisch erfolgt.

9. Verfahren nach Anspruch 1 bis 8, gekennzeichnet dadurch, dass die Zugabe der Nucleinsäureglycosylase für den späteren Abbau der Starter-Oligonucleotid-Sequenzen und deren Komplementärsequenzen zum Reaktionsgemisch der ursprünglichen Amplifikation erfolgt.

10. Verfahren nach Anspruch 1 bis 8, gekennzeichnet dadurch, dass der Abbau der Starter-Oligonucleotid-Sequenzen und deren Komplementärsequenzen durch Zugabe der Nucleinsäureglycosylase zum Reaktionsgemisch vor Beginn der neuen Amplifikation erfolgt.

11. Oligonucleotide zum Einsatz in einem Verfahren nach Anspruch 1 bis 10 gekennzeichnet dadurch, dass sie mindestens ein Derivat von Nucleotidresten enthalten, deren Stickstoffbase nach Transformation durch eine Nucleinsäureglycosylase abgespalten werden kann.

12. Oligonucleotide nach Anspruch 11, gekennzeichnet dadurch, dass es sich bei den in die Starter-Oligonucleotide eingebauten Derivaten von Nucleotidresten um 5-(2-(1,3-Dioxanyl))-uracil, 5-(2-Nitrobenzoxy)methylcytosin, 5-(4-Methoxy-2-nitrobenzoxy)methylcytosin, 5-(4,5-Dimethoxy-2-nitrobenzoxy)methylcytosin [= 5-(6-Nitroveratryloxy)methylcytosin], 5-(2-Nitrobenzoxy)methyluracil, 5-(4-Metho-xy-2-nitrobenzoxy)methyluracil, 5-(4,5-Dimethoxy-2-nitrobenzoxy)methyluracil [= 5-(6-Nitroveratryloxy)methyluracil], 5-(2-(4-(2-Nitrophenyl)-1,3-dioxola-nyl))-methyluracil, 6-(2-Nitrobenzoxy)purin, 6-(4-Methoxy-2-nitrobenzoxy)purin, 6-(4,5-Dimethoxy-2-nitrobenzoxy)purin [= 5-(6-Nitroveratryloxy)purin] handelt.

13. Oligonucleotide nach Anspruch 11, gekennzeichnet dadurch, dass die Transformation des mindestens einen Derivates von Nucleotidresten chemisch, insbesondere photolytisch, erfolgt.

14. Oligonucleotide nach Anspruch 10, gekennzeichnet dadurch, dass die Transformation des mindestens einen Derivates von Nucleotidresten biochemisch, insbesondere enzymatisch erfolgt.
